**Europäisches Patentamt**

**European Patent Office**

**Office européen des brevets**

(19)

(11) Veröffentlichungsnummer : **0 295 199 B1**

(12) **EUROPÄISCHE PATENTSCHRIFT**

(45) Veröffentlichungstag der Patentschrift :
**17.04.91 Patentblatt 91/16**

(51) Int. Cl.$^5$ : **A61F 2/32**

(21) Anmeldenummer : **88730133.1**

(22) Anmeldetag : **13.06.88**

(54) Hüftgelenksprothese mit einem zylindrischen Schaftteil.

(30) Priorität : **12.06.87 DE 8708500 U**

(43) Veröffentlichungstag der Anmeldung :
**14.12.88 Patentblatt 88/50**

(45) Bekanntmachung des Hinweises auf die
Patenterteilung :
**17.04.91 Patentblatt 91/16**

(84) Benannte Vertragsstaaten :
**AT BE CH DE ES FR GB IT LI NL SE**

(56) Entgegenhaltungen :
**EP-A- 0 086 879**
**EP-A- 0 179 626**
**EP-A- 0 204 919**

(73) Patentinhaber : **MECRON MEDIZINISCHE
PRODUKTE GMBH
Nunsdorfer Ring 23-29
W-1000 Berlin 48 (DE)**

(72) Erfinder : **Kranz, Curt
Kufsteiner Strasse 12
W-1000 Berlin 62 (DE)**

(74) Vertreter : **Christiansen, Henning, Dipl.-Ing.
Patentanwalt CHRISTIANSEN Pacelliallee
43/45
W-1000 Berlin 33 (DE)**

# Beschreibung

Die Erfindung betrifft eine Hüftgelenksprothese der im Oberbegriff des Anspruchs 1 angegebenen Art sowie eine Vorrichtung zur Durchführung des Verfahrens.

Eine Hüftgelenksprothese dieser Art ist aus der europäischen Patentanmeldung EP-A-0 179 626 bekannt.

Bei dieser Prothese ist der konische Schaft jeweils durch Ansätze verlängerbar, welche auf das Ende des konischen Schaftes des den Ausleger mit der Gelenkkugel tragenden Teils aufschiebbar sind. Nachteilig ist dabei, daß insbesondere bei größeren Schaftlängen Instabilitäten entstehen können, weil das Überlappen aufeinanderfolgender Elemente auf den Bereich des Konus beschränkt ist. Bei mehreren Verlängerungen wären (entsprechend den bekannten "Baukastenprothesen") mehrere Verlängerungselemente mit den dazugehörigen Konusverbindungen notwendig. Darüber hinaus ist die Masse des Schaftes – insbesondere bei größeren Längen – beträchtlich.

Besonders vorteilhaft bei der erfindungsgemäßen Prothese ist, daß dasjenige Bauteil, welches zur Herstellung ein Schmiedegesenk erfordert, für alle Prothesen eines Systems mit unterschiedlichen Schaftlängen identisch ausgeführt sein kann, während die weiteren Teile bevorzugt aus einem Rohrmaterial als Halbzeug gefertigt werden können.

Der im kennzeichnenden Teil des Anspruchs 1 angegebenen Erfindung liegt die Aufgabe zugrunde, bei einer Hüftgelenksprothese der eingangs genannten Gattung, die bei unter Austausch mit verhältnismäßig geringem Herstellungsaufwand herstellbaren Teilen ein variables System von Prothesen mit großer Steifigkeit und kleiner Masse ermöglicht.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen :

Figur 1 ein Ausführungsbeispiel der erfindungsgemäßen Prothese in Explosionsdarstellung und

Figur 2 das Ausführungsbeispiel gemäß Figur 1 zusammengefügt im Querschnitt dargestellt.

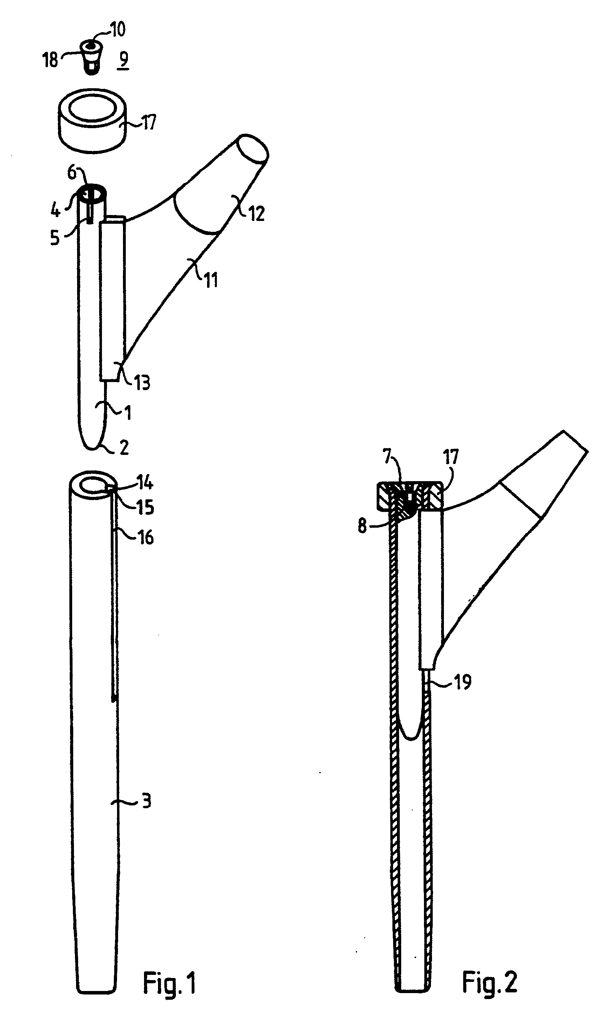

Bei dem in Figur 1 dargestellten Ausführungsbeispiel weist ein erstes Teil 1 einen zylindrischen, konischen oder in sonstiger Weise prismatischen Bereich auf, der an seinem gelenkfernen Ende 2 eine Verjüngung mit Verrundung aufweist, welche ein leichtes Einfügen dieses Teils 1 in ein weiteres Schaftteil 3 ermöglicht. An seinem oberen (gelenknahen) Ende weist das Teil 1 eine Bohrung 4 auf und ist dort mit Schlitzen 5 und 6 versehen. Die Bohrung weist in ihrem oberen Bereich eine konische Erweiterung 7 und in ihrem tiefer gelegenen Endbereich ein Innengewinde 8 auf (Figur 2). Auf diese Weise ist durch eine Schraube 9 mit einem Innensechskant 10, welche in die Gewindebohrung 8 einfügbar ist, ein Aufspreizen des oberen Endes des Teils 1 möglich, wenn die Schraube angezogen wird. Der Schaftteil 1 weist des weiteren einen Ausleger 11 auf, der einen Konus 12 trägt, auf den eine Gelenkkugel aufsteckbar ist (Die Gelenkkugel ist in den Figuren nicht dargestellt – sie ist entsprechend den individuellen Anforderungen des Patienten aus einem Sortiment unterschiedlicher Größen auswählbar).

Der Ausleger 11 geht in seinem schaftnahen Teil in einen Bereich 13 über, der in tangentialer Richtung (bezogen auf den Schaft) eine relativ geringe Erstreckung aufweist, während die Ausdehnung in Schaftrichtung demgegenüber wesentlich größer ist. Der Wurzelbereich des Auslegers geht damit im schaftnahen Teil in einen Körper über, welcher zwei einander gegenüberliegende planparallele Flächen aufweist, die die Seitenflächen 14 und 15 eines Schlitzes 16, der in dem Teil 3 in dessen Längsrichtung erzeugt ist, berühren bzw. daran flächig anliegen und somit eine Führung bilden.

Die Abmessung des Elementes 13, die in Schaftrichtung größer ist als die der längeren Halbachse der parallel zur Schaftlängsachse ausgerichteten (elliptischen) Schnittebene des im wesentlichen zylindrischen Querschnitts des Auslegers. Die in Bezug auf den Schaft tangentiale Erstreckung des Elementes 13 ist hingegen kleiner als die kleine Halbachse der entsprechenden Ellipse.

Die genannte Ellipse ist in der Zeichnung nicht eingezeichnet, sie ist jedoch aufgrund der vorstehenden Angaben definiert.

Die Befestigung des Teils 1 in dem Teil 3 erfolgt nun dadurch, daß nach dem Einschieben des Schaftteiles 1 in die Hülse 3, wobei das Element 13 in den Schlitz 16 gelangt, ein Spannring 17 über das obere Ende des Teils 3 geschoben wird. Anschließend wird die Schraube 9 in die Bohrung 4 eingesetzt und in das Innengewinde 8 eingedreht. Der konisch sich erweiternde Kopfbereich 18 der Schraube 9 (Außenkonus) tritt mit dem konischen Bereich 7 (Innenkonus) der Bohrung in Wechselwirkung und spreizt den mit Schlitzen 5 und 6 versehenden Bereich des Teils 1 auf, so daß der obere hülsenförmige Bereich des Teils 3 gegen die Innenwandung des Rings 17 gepreßt und somit eine Spannverbindung erzeugt wird.

Dadurch, daß der Schlitz 16 eine Überlänge (Bereich 19 in Figur 2) aufweist, ist das Teil 1 in der Hülse 3 in der Höhe justierbar – auch noch nachdem das Teil 3 bereits im Markraum befestigt ist, da die Fixierung (Schraube 9) von oben her zugänglich ist. Bei einer möglicherweise erforderlich werdenden Reoperation ist auch nach Entfernung des Teils 1 das Schaftteil 3 noch von oben her zugänglich und kann leicht ergriffen und wegen seiner geradlinigen zylindrischen Form ohne besondere Schwierigkeiten entfernt werden und durch ein entsprechendes längeres Element ersetzt werden.

Bei Varianten der Erfindung im Sinne eines Prothesensystems kann als Schaftteil 3 bevorzugt auch ein in Längsrichtung durchgehend geschlitzter Küntscher-Nagel verwendet werden, so daß die Vorteile der Markraum-Nagelung mit derjenigen der hier dargestellten Hüftgelenksprothese verbunden werden können.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Ansprüche

1. Hüftgelenksprothese mit einem in der Nähe des gelenknahen Endes gelegenen im wesentlichen zylindrischen ersten Schaftteil(1), der einen in Richtung dieses Endes schräg gestellten seitlichen Ausleger (11) für die Gelenkkugel aufweist, wobei der Ausleger (11) in seinem an den Schaft angrenzenden Bereich in Richtung des Schafts (1) eine wesentlich größere Abmessung hat als tangential dazu und wobei ein auf den den Ausleger (11) tragenden ersten Schaftteil (1) ein zweiter Schaftteil (3) aufsteckbar ist, der den ersten Schaftteil 1 in sich vom Gelenkende entfernender Richtung überragt, **dadurch gekennzeichnet,** daß der zweite Schaftteil (3) im wesentlichen vollständig über den den Ausleger (11) tragenden ersten Schaftteil (1) schiebbar und am gelenknahen Ende des ersten Schaftteils (1) fixierbar ist, wobei der Ausleger sich durch eine Einschlitzung (16) des zweiten Schaftteils (3) in der Weise hindurch erstreckt, daß der Auslegeransatz (13) sich im wesentlichen in der Einschlitzung (16) befindet.

2. Hüftgelenksprothese nach Anspruch 1, **dadurch gekennzeichnet,** daß zur Aufrechterhaltung der Verbindung der beiden Schaftteile (1 und 3) eine mit dem zweiten Schaftteil (3) verbundene Sicherung vorgesehen ist, die sich oberhalb des Auslegers (11) quer zur Längsachse der Schaftteile (1 und 3) erstreckt.

3. Hüftgelenksprothese nach einem der vorangehenden Ansprüche, **dadurch gekennzeichnet,** daß der zweite Schaftteil (3) durchgehend hohl ausgebildet ist.

4. Hüftgelenksprothese nach einem der vorangehenden Ansprüche **dadurch gekennzeichnet,** daß das gelenkferne Ende (2) des zweiten Schaftteil (3) sich konisch verjüngt.

5. Hüftgelenksprothese nach Anspruch 4, **dadurch gekennzeichnet,** daß der zweite Schaftteil (3) als Blechprofil ausgebildet ist.

6. Hüftgelenksprothese nach einem der vorangehenden Ansprüche, **dadurch gekennzeichnet,** daß der zweite Schaftteil (3) durchgehend geschlitzt in Form eines Küntscher-Nagels ausgebildet ist.

7. Hüftgelenksprothese nach einem der Ansprüche 2 bis 6, **dadurch gekennzeichnet,** daß die Sicherung einen das gelenknahe Ende (2) des zweiten Schaftteils (3) umgebenden Ring (17) aufweist.

8. Hüftgelenksprothese nach Anspruch 7, **dadurch gekennzeichnet,** daß der Ring (17) durch eine in das erste Schaftteil (1) eingreifende Schraube (9) gesichert ist.

9. Hüftgelenksprothese nach Anspruch 8, **dadurch gekennzeichnet,** daß die Schraube (9) einen sich in Richtung zunehmender Entfernung vom Gewinde konisch erweiternden Kopf (18) aufweist, der in eine entsprechend geformte Ausnehmung (4) des Endes des ersten Schaftteils (1) eingreift, welches insbesondere unter Verwendung eines Schlitzes (5), aufspreizbar ausgebildet ist.

## Claims

1. Hip joint prosthesis with an essentially cylindrical first shaft part (1) situated in the vicinity of the end near the joint, which shaft part (1) has a lateral extension arm (11) inclined in the direction of this end and intended for the ball of the joint, the area of the extension arm (11) adjoining the shaft having a substantially greater dimension in the direction of the shaft (1) than tangentially thereto, and a second shaft part (3) being applied to the first shaft part (1) bearing the extension arm (11), which second shaft part (3) projects beyond the first shaft part (1) in the direction away from the joint end, characterised in that the second shaft part (3) can be pushed essentially completely over the first shaft part (1) bearing the extension arm (11) and can be secured at that end of the first shaft part (1) near the joint, the extension arm extending through a slot (16) in the second shaft part (3) in such a way that the shoulder (13) of the extension arm is located essentially in the slot (16).

2. Hip joint prosthesis according to Claim 1, characterised in that, in order to maintain the connection of the two shaft parts (1 and 3), a securing piece connected to the second shaft part (3) is provided, this securing piece extending above the extension arm (11) transversely to the longitudinal axis of the shaft parts (1 and 3).

3. Hip joint prosthesis according to one of the preceding claims, characterised in that the second shaft part (3) is designed continuously hollow.

4. Hip joint prosthesis according to one of the preceding claims, characterised in that that end of the second shaft part (3) remote from the joint tapers conically.

5. Hip joint prosthesis according to Claim 4, characterised in that the second shaft part (3) is designed as a sheet metal profile.

6. Hip joint prosthesis according to one of the preceding claims, characterised in that the second shaft part (3) is designed with a continuous slot in the form of a Küntscher nail.

7. Hip joint prosthesis according to one of Claims 2 to 6, characterised in that the securing piece has a ring (17) surrounding that end of the second shaft part (3) near the joint.

8. Hip joint prosthesis according to Claim 7, characterised in that the ring (17) is fastened by means of a screw (9) engaging in the first shaft part (1).

9. Hip joint prosthesis according to Claim 8, characterised in that the screw (9) has a head (18) which widens conically in the direction of increasing distance from the thread and which engages in a correspondingly formed recess (4) in the end of the first shaft part (1) which is designed in such a way that it can spread open, in particular by means of a slit (5).

**Revendications**

1. Prothèse de hanche avec une première pièce de tige (1) essentiellement cylindrique logée à proximité de l'extrémité voisine de l'articulation, qui présente un bras (11) placé en biais vers cette extrémité, pour la sphère de l'articulation, dans laquelle le bras (11) dans sa zone limitrophe de la tige dirigée vers la tige (1) présente une dimension nettement plus grande qu'en sens tangentiel, et
dans laquelle on peut enfiler une deuxième pièce de tige (3) sur la première pièce de tige (1) portant le bras (11), qui s'étend dans le sens opposé à l'extrémité d'articulation et dépasse la première pièce de tige (1), caractérisé en ce que,
la deuxième pièce de tige (3) peut coulisser esentiellement complètement sur la première pièce de tige (1) portant le bras (11) et peut être fixée sur l'extrémité proche de l'articulation de la première pièce de tige (1), le bras s'étendant dans une fente (16) de la deuxième partie de tige (3) de telle manière que la racine du bras (13) se trouve essentiellement dans la fente (16).

2. Prothèse de la hanche selon la revendication 1, caractérisée en ce que, pour maintenir la liaison des deux pièces de tige (1 et 3), on a prévu une sécurité liée à la deuxième pièce de tige (3), qui s'étend au-dessus du bras (11) transversalement à l'axe longitudinal des pièces de tige (1 et 3).

3. Prothèse de la hanche selon l'une des revendications précédentes, caracterisée en ce que la deuxième pièce de tige (3) est creuse sur toute sa longueur.

4. Prothèse selon l'une des revendications précédentes, caractérisée en ce que l'extrémité (2) opposée à l'articulation de la deuxième pièce de tige (3) se rétrécie en cône.

5. Prothèse de hanche selon la revendication 4, caractérisée en ce que la deuxième pièce de tige (3) est en profilé de tôle.

6. Prothèse de hanche selon l'une des revendications précédentes, caractérisée en ce que la deuxième pièce de tige (3) est fendue sur toute la longueur en forme de clou de Küntscher.

7. Prothèse selon l'une des revendications 2 à 6, caractérisée en ce que la sécurité présente un anneau (17) entourant l'extrémité (2) proche de l'articulation de la deuxième pièce de tige (3).

8. Prothèse de hanche selon la revendication 7, caractérisée en ce que l'anneau (17) est fixé par une vis (9) en prise dans la première pièce de tige (1).

9. Prothèse selon la revendication 8, caractérisée en ce que la vis (9) présente une tête (18) s'évasant en cône en s'éloignant du filetage, qui est en prise dans un évidement (4) de forme correspondante de l'extrémité de la première pièce de tige (1), qui peut s'élargir notamment en utilisant une fente (5).

Fig.1

Fig.2